# EUROPEAN PATENT APPLICATION

(11) **EP 3 692 838 A1**
(43) Date of publication of application: **12.08.2020**
(21) Application number: 19155877.4
(22) Date of filing: 07.02.2019
(51) Int. Cl.: A24F 47/00, A61M 15/06, A61M 11/04, A61M 11/00

(54) **SMOKING SUBSTITUTE APPARATUS**

(71) Applicant: NERUDIA LIMITED, Liverpool L24 9HP (GB)
(72) Inventor: The designation of the inventor has not yet been filed
(74) Representative: Mewburn Ellis LLP

(57) **Abstract**

A flavour delivery article for a smoking substitute device, the flavour delivery article comprising:
a flavourant source comprising a fragranced fluid; and
a flavour generator configured to agitate the fragranced fluid to thereby release the fragranced fluid from the flavour delivery article for olfactory flavour delivery.

## Description

### Field of the Invention

The present invention relates to a smoking substitute apparatus and, in particular, a smoking substitute apparatus that is able to deliver flavour to a user.

### Background

The smoking of tobacco is generally considered to expose a smoker to potentially harmful substances. It is generally thought that a significant amount of the potentially harmful substances are generated through the heat caused by the burning and/or combustion of the tobacco and the constituents of the burnt tobacco in the tobacco smoke itself.

Combustion of organic material such as tobacco is known to produce tar and other potentially harmful byproducts. There have been proposed various smoking substitute systems in order to avoid the smoking of tobacco.

Such smoking substitute systems can form part of nicotine replacement therapies aimed at people who wish to stop smoking and overcome a dependence on nicotine.

Smoking substitute systems include electronic systems that permit a user to simulate the act of smoking by producing an aerosol (also referred to as a "vapour") that is drawn into the lungs through the mouth (inhaled) and then exhaled. The inhaled aerosol typically bears nicotine and/or a flavourant without, or with fewer of, the odour and health risks associated with traditional smoking.

In general, smoking substitute systems are intended to provide a substitute for the rituals of smoking, whilst providing the user with a similar experience and satisfaction to those experienced with traditional smoking and with combustible tobacco products.

The popularity and use of smoking substitute systems has grown rapidly in the past few years. Although originally marketed as an aid to assist habitual smokers wishing to quit tobacco smoking, consumers are increasingly viewing smoking substitute systems as desirable lifestyle accessories. There are a number of different categories of smoking substitute systems, each utilising a different smoking substitute approach.

One approach is the so-called "vaping" approach, in which a vaporisable liquid, typically referred to (and referred to herein) as "e-liquid", is heated by a heating device (referred to herein as an electronic cigarette or "e-cigarette" device) to produce an aerosol vapour which is inhaled by a user. The e-liquid typically includes a base liquid as well as nicotine and/or a flavourant. The resulting vapour therefore also typically contains nicotine and/or a flavourant. The base liquid may include propylene glycol and/or vegetable glycerine.

A typical e-cigarette device includes a mouthpiece, a power source (typically a battery), a tank for containing e-liquid, as well as a heating device. In use, electrical energy is supplied from the power source to the heating device, which heats the e-liquid to produce an aerosol (or "vapour") which is inhaled by a user through the mouthpiece.

E-cigarettes can be configured in a variety of ways. For example, there are "closed system" vaping smoking substitute systems, which typically have a sealed tank and heating element. The tank is prefilled with e-liquid and is not intended to be refilled by an end user. One subset of closed system vaping smoking substitute systems include a main body which includes the power source, wherein the main body is configured to be physically and electrically coupled to a consumable including the tank and the heating element. In this way, when the tank of a consumable has been emptied, that consumable is disposed of. The main body can be reused by connecting it to a new, replacement, consumable. Another subset of closed system vaping smoking substitute systems are completely disposable, and intended for one-use only.

There are also "open system" vaping smoking substitute systems which typically have a tank that is configured to be refilled by a user. In this way the entire device can be used multiple times.

An example vaping smoking substitute system is the myblu™ e-cigarette. The myblu™ e-cigarette is a closed system which includes a main body and a consumable. The main body and consumable are physically and electrically coupled together by pushing the consumable into the main body. The main body includes a rechargeable battery. The consumable includes a mouthpiece, a sealed tank which contains e-liquid, as well as a heater, which for this device is a heating filament coiled around a portion of a wick. The wick is partially immersed in the e-liquid, and conveys e-liquid from the tank to the heating filament. The device is activated when a microprocessor on board the main body detects a user inhaling through the mouthpiece. When the device is activated, electrical energy is supplied from the power source to the heating device, which heats e-liquid from the tank to produce a vapour which is inhaled by a user through the mouthpiece.

For a smoking substitute device it is desirable to deliver nicotine into the user's lungs, where it can be absorbed into the bloodstream. As explained above, in the so-called "vaping" approach, e-liquid is heated by a heating device to produce an aerosol vapour which is inhaled by a user. Many e-cigarettes also deliver flavour to the user to enhance the experience. In such e-cigarettes, flavour compounds are contained in the e-liquid that is heated. However, toxicology restrictions are placed on the amount of flavour that can be contained in the e-liquid, and this can result in some e-liquid flavours delivering a weak and underwhelming taste sensation to consumers in the pursuit of safety. Further, there is a view that providing a flavourant as part of the e-liquid, such that the flavourant is vaporised with the e-liquid, may be disadvantageous.

There may be a need for improved design of smoking substitute systems, in particular in regards to the delivery of flavour to a user.

The present disclosure has been devised in the light of the above considerations.

### Summary of the Invention

At its most general, the present invention relates to a flavour delivery article for a smoking substitute device.

According to a first aspect there is provided a flavour delivery article for a smoking substitute device, the flavour delivery article comprising a flavourant source comprising a fragranced fluid, and a flavour generator configured to agitate the fragranced fluid to thereby release the fragranced fluid from the flavour delivery article for olfactory flavour delivery.

The agitation (i.e. excitement) of the fragranced fluid leads to the release of the fragranced fluid from the flavour delivery article. The fragranced fluid may be released as a mist, aerosol or spray, for example.

Advantageously, a flavour can be sensed through the nose of a user rather than within the mouth of the user, by inhaling the fragranced fluid through the nose. In other words, the effect of flavour can be provided through the sense of smell rather than the sense of taste. In this way, the delivery of flavour is separate from delivery of e-liquid vapour in the smoking substitute device, which may or may not contain nicotine. This can affect and/or increase the amount, strength or intensity of flavour sensed by a user.

Preferably, the flavour generator is configured to agitate the fragranced fluid in response to a user input.

Accordingly, the delivery of flavour to a user depends on an action of a user. The flavour delivery article may only release the fragranced liquid when a user indicates that they require flavour. Accordingly, the flavour delivery article may not continuously release flavour. In this way, a user can choose whether they want flavour delivery during use of the smoking substitute device (i.e. during vaping).

The user input could be an action of a user, such as pushing a button or dial. Alternatively, the user input could be the act of drawing on (i.e. vaping) the smoking substitute device itself.

The flavour generator may be electrically powered.

Thus, the flavour generator may be powered by an electrical power source, such as a battery. The battery may be rechargeable. The user input may result in a change to the supply of power to the flavour generator to activate the flavour generator.

An amount of electrical power supplied to the flavour generator may be adjustable to modify the amount of fragranced fluid released from the flavour delivery article.

In this way, the strength, intensity and/or amount of flavour received by a user can be adjusted. The adjustment may be based on a user's preference.

Specifically, the amount of electrical power supplied to the flavour generator may be controllable by a user of the smoking substitute device to increase and/or decrease the amount of fragranced fluid released from the flavour delivery article, and thereby the amount of flavour received by a user. The user may be able to adjust the amount of electrical power supplied to the flavour generator by a button, dial or slider on the flavour delivery article and/or on the smoking substitute device, for example.

The flavour generator may be a piezoelectric motor.

A piezoelectric motor is an electromechanical device that uses the converse piezoelectric effect to convert electrical energy into mechanical energy. The piezoelectric motor is configured to receive an electrical input in the form of a high-frequency signal and convert this signal into vibrationary motion at the same frequency. This high-frequency oscillation agitates the fragranced fluid in the flavourant source, thereby releasing a mist of the fragranced fluid from the flavourant source.

The piezoelectric motor may comprise a piezoelectric element and a resonating surface, the resonating surface may be positioned adjacent to the flavourant source, and the piezoelectric element may be configured to vibrate the resonating surface upon receipt of an electrical signal, the resonating surface thereby providing a force on the flavourant source to agitate the fragranced fluid.

The piezoelectric element may comprise a piezoelectric material such as a ceramic material. The resonating surface may form a portion of the piezoelectric element, or may be formed from another element of the piezoelectric motor.

Optionally, the flavourant source may be an absorbable pad and the fragranced fluid may be a fragranced liquid absorbed into the pad.

In this way, the flavour generator (e.g. the piezoelectric motor) may agitate the fragranced liquid absorbed in the pad. This excitement of the fragranced liquid in the absorbable pad may result in the release of a mist, spray or aerosol of fragranced liquid from the pad, and therefore from the flavour delivery article. The mist of fragranced liquid can then be inhaled through the nose of a user to deliver flavour.

Alternatively, the flavourant source may be a pocket, and the fragranced fluid may be a fragranced liquid contained in the pocket.

In this way, the flavour generator (e.g. the piezoelectric motor) may agitate the fragranced liquid contained in the pocket which in turn may release the fragranced liquid from the pocket, and therefore from the flavour delivery article. The fragranced liquid can then be inhaled through the nose of a user to deliver flavour.

The pocket may comprise an outlet mister, the outlet mister being configured to release a mist of the fragranced liquid upon agitation of the fragranced liquid by the flavour generator.

The outlet mister may release the mist of fragranced fluid in a specified particle size, such that the fragranced fluid can be easily and efficiently received by a user's nose for olfactory flavour delivery.

Accordingly, a mist, spray or aerosol of fragranced liquid may be released from the pocket, and therefore from the flavour delivery article. The mist of fragranced liquid can then be inhaled through the nose of a user to deliver flavour.

The flavour delivery article may further comprise attaching means for attaching the flavour delivery article to a smoking substitute device.

The attaching means may be a clip, for example.

Accordingly, the flavour delivery article may be easily attachable and detachable from a smoking substitute device, such as an electronic cigarette. A user or the smoking substitute device can choose whether they would like the delivery of flavour by attaching or detaching the flavour delivery article to the smoking substitute device. The flavour delivery article may be attachable to a smoking substitute apparatus, such as a consumable, or a main body of the smoking substitute device.

Furthermore, a user of the smoking substitute device is able to change the flavour delivered simply by detaching a flavour delivery article having a first flavour (e.g. a fruit flavour) for a flavour delivery article having a second flavour (i.e. a spiced flavour). Further still, the flavour delivery article can be easily replaced once all of the fragranced fluid has been used.

Optionally, the flavourant source may be refillable with fragranced fluid in order to replenish diminished amounts of fragranced fluid.

According to a second aspect there is provided a smoking substitute apparatus comprising a tank for containing a vaporisable e-liquid, a heating element for heating the vaporisable e-liquid, and the flavour delivery article of the first aspect.

The smoking substitute apparatus may be a consumable (i.e. a "pod").

According to a third aspect there is provided a smoking substitute device comprising a tank for containing a vaporisable e-liquid, a heating element for heating the vaporisable e-liquid, an electrical power source for providing electrical power to the heating element, and the flavour delivery article of the first aspect.

The electrical power source may be a battery, for example.

In this way, the smoking substitute device can supply vaporised e-liquid to the mouth of a user, and fragranced fluid to the nose of a user for olfactory flavour delivery. Accordingly, the delivery of flavour is separate from delivery of e-liquid vapour, which may or may not contain nicotine. This can affect and/or increase the amount and/or strength of flavour sensed by a user.

Preferably, the smoking substitute device may have opposing ends, wherein one end of the smoking substitute device defines a mouthpiece, and the flavour delivery article is configured to release the fragranced fluid generally towards the mouthpiece.

Additionally, the flavour delivery article may be positioned, arranged and/or orientated such that, in use, the fragranced fluid is released towards the nose of a user.

By releasing the fragranced fluid generally towards the mouthpiece, the fragranced fluid is directed towards a user's nose during vaping. In this way, the user can receive a stronger and/or more intense flavour as more of the released fragranced fluid can be received by the user's nose.

When the flavour generator is a piezoelectric motor, the electrical power source may be electrically connectable to the piezoelectric motor to provide electrical power to the piezoelectric motor.

Thus, the electrical power source can power both the heating element and the piezoelectric motor. Therefore, whenever the heating element is operative to provide e-liquid vapour to a user, the flavour delivery article is also activated.

Alternatively, the piezoelectric motor may be electrically powered by a second power source which is different from the power source for providing power to the heating element.

The smoking substitute device may further comprise a controller for controlling the supply of electrical power to the heating element and/or the piezoelectric motor.

Accordingly, the supply of power to the piezoelectric motor can be started, stopped and/or varied, which in turn starts, stops and/or varies the amount of fragranced fluid released from the flavour delivery article for olfactory delivery to a user.

The smoking substitute device may further comprise a puff sensor operatively connectable to the controller so as to be able to provide a signal to the controller that is indicative of a detected puff by a user, wherein the controller may be configured to instruct the supply of electrical power to the heating element and/or the piezoelectric motor in response to the receipt of the signal.

In this way, the piezoelectric motor is automatically activated when a user inhales (i.e. draws on the smoking substitute device), so that flavour is automatically delivered when a user inhales. The pressure sensor may detect a drop in pressure when the user inhales which thereby activates the piezoelectric motor.

The fragranced fluid may comprise a flavourant. The term "flavourant" is used to describe a compound or combination of compounds that provide flavour and/or aroma. For example, the flavourant may be configured to interact with a sensory receptor of a user (such as an olfactory or taste receptor). The flavourant may include one or more volatile substances.

The flavourant may be provided in liquid, gel or gaseous form. The flavourant may be natural or synthetic. For example, the flavourant may include menthol, liquorice, chocolate, fruit flavour (including e.g. citrus, cherry etc.), vanilla, spice (e.g. ginger, cinnamon) and tobacco flavour. The flavourant may be evenly dispersed or may be provided in isolated locations and/or varying concentrations.

The smoking substitute apparatus may be in the form of a consumable. The consumable may be configured for engagement with a main body (i.e. so as to form a closed smoking substitute system). For example, the consumable may comprise components of the system that are disposable, and the main body may comprise non-disposable or non-consumable components (e.g. power supply, controller, sensor, etc.) that facilitate the delivery of aerosol by the consumable. In such an embodiment, the aerosol former (e.g. e-liquid) may be replenished by replacing a used consumable with an unused consumable.

Alternatively, the smoking substitute apparatus may be a non-consumable apparatus (e.g. that is in the form of an open smoking substitute system). In such embodiments an aerosol former (e.g. e-liquid) of the system may be replenished by re-filling e.g. a reservoir of the smoking substitute apparatus with the aerosol former (rather than replacing a consumable component of the apparatus).

In light of this, it should be appreciated that some of the features described herein as being part of the smoking substitute apparatus may alternatively form part of a main body for engagement with the smoking substitute apparatus (i.e. when the smoking substitute apparatus is in the form of a consumable).

Where the smoking substitute apparatus is in the form of a consumable, the main body and the consumable may be configured to be physically coupled together. For example, the consumable may be at least partially received in a recess of the main body, such that there is an interference fit between the main body and the consumable. Alternatively, the main body and the consumable may be physically coupled together by screwing one onto the other, or through a bayonet fitting.

Thus, the smoking substitute apparatus may comprise one or more engagement portions for engaging with a main body. In this way, one end of the smoking substitute apparatus may be coupled with the main body, whilst an opposing end of the smoking substitute apparatus may define a mouthpiece of the smoking substitute system.

The smoking substitute apparatus may comprise a reservoir configured to store an aerosol former, such as an e-liquid. The e-liquid may, for example, comprise a base liquid and e.g. nicotine. The base liquid may include propylene glycol and/or vegetable glycerine. The e-liquid may be flavourless (i.e. not contain any flavourants) and may consist solely of a base liquid of propylene glycol and/or vegetable glycerine and nicotine.

The reservoir may be in the form of a tank. At least a portion of the tank may be translucent. For example, the tank may comprise a window to allow a user to visually assess the quantity of e-liquid in the tank. A housing of the smoking substitute apparatus may comprise a corresponding aperture (or slot) or window that may be aligned with a translucent portion (e.g. window) of the tank. The reservoir may be referred to as a "clearomizer" if it includes a window, or a "cartomizer" if it does not.

The smoking substitute apparatus may comprise a passage for fluid flow therethrough. The passage may extend through (at least a portion of) the smoking substitute apparatus, between openings that may define an inlet and an outlet of the passage. The outlet may be at a mouthpiece of the smoking substitute apparatus. In this respect, a user may draw fluid (e.g. air) into and through the passage by inhaling at the outlet (i.e. using the mouthpiece). The passage may be at least partially defined by the tank. The tank may substantially (or fully) define the passage. In this respect, the tank may surround the passage.

The smoking substitute apparatus may comprise an aerosol-generator. The aerosol generator may comprise a wick. The aerosol generator may further comprise a heater. The wick may comprise a porous material. A portion of the wick may be exposed to fluid flow in the passage. The wick may also comprise one or more portions in contact with liquid stored in the reservoir. For example, opposing ends of the wick may protrude into the reservoir and a central portion (between the ends) may extend across the passage so as to be exposed to fluid flow in the passage. Thus, fluid may be drawn (e.g. by capillary action) along the wick, from the reservoir to the exposed portion of the wick.

The heater may comprise a heating element, which may be in the form of a filament wound about the wick (e.g. the filament may extend helically about the wick). The filament may be wound about the exposed portion of the wick. The heating element may be electrically connected (or connectable) to a power source. Thus, in operation, the power source may supply electricity to (i.e. apply a voltage across) the heating element so as to heat the heating element. This may cause liquid stored in the wick (i.e. drawn from the tank) to be heated so as to form a vapour and become entrained in fluid flowing through the passage. This vapour may subsequently cool to form an aerosol in the passage.

The smoking substitute apparatus (or main body engaged with the smoking substitute apparatus) may comprise a power source. The power source may be electrically connected (or connectable) to a heater of the smoking substitute apparatus (e.g. when engaged with the main body). The power source may be a battery (e.g. a rechargeable battery). A connector in the form of e.g. a USB port may be provided for recharging this battery.

When the smoking substitute apparatus is in the form of a consumable, the smoking substitute apparatus may comprise an electrical interface for interfacing with a corresponding electrical interface of the main body. One or both of the electrical interfaces may include one or more electrical contacts. Thus, when the main body is engaged with the consumable, the electrical interface may be configured to transfer electrical power from the power source to a heater of the consumable.

The electrical interface may also be used to identify the smoking substitute apparatus (in the form of a consumable) from a list of known types. For example, the consumable may have a certain concentration of nicotine and the electrical interface may be used to identify this. The electrical interface may additionally or alternatively be used to identify when a consumable is connected to the main body.

Again, where the smoking substitute apparatus is in the form of a consumable, the main body may comprise an interface, which may, for example, be in the form of an RFID reader, a barcode or QR code reader. This interface may be able to identify a characteristic (e.g. a type) of a consumable engaged with the main body. In this respect, the consumable may include any one or more of an RFID chip, a barcode or QR code, or memory within which is an identifier and which can be interrogated via the interface.

The smoking substitute apparatus or main body may comprise a controller, which may include a microprocessor. The controller may be configured to control the supply of power from the power source to the heater of the smoking substitute apparatus (e.g. via the electrical contacts). A memory may be provided and may be operatively connected to the controller. The memory may include non-volatile memory. The memory may include instructions which, when implemented, cause the controller to perform certain tasks or steps of a method.

The main body or smoking substitute apparatus may comprise a wireless interface, which may be configured to communicate wirelessly with another device, for example a mobile device, e.g. via Bluetooth®. To this end, the wireless interface could include a Bluetooth® antenna. Other wireless communication interfaces, e.g. WiFi®, are also possible. The wireless interface may also be configured to communicate wirelessly with a remote server.

A puff sensor may be provided that is configured to detect a puff (i.e. inhalation from a user). The puff sensor may be operatively connected to the controller so as to be able to provide a signal to the controller that is indicative of a puff state (i.e. puffing or not puffing). The puff sensor may, for example, be in the form of a pressure sensor or an acoustic sensor. That is, the controller may control power supply to the heater of the consumable in response to a puff detection by the sensor. The control may be in the form of activation of the heater in response to a detected puff. That is, the smoking substitute apparatus may be configured to be activated when a puff is detected by the puff sensor. When the smoking substitute apparatus is in the form of a consumable, the puff sensor may form part of the consumable or the main body.

The invention includes the combination of the aspects and preferred features described except where such a combination is clearly impermissible or expressly avoided.

### Summary of the Figures

So that the invention may be understood, and so that further aspects and features thereof may be appreciated, embodiments illustrating the principles of the invention will now be discussed in further detail with reference to the accompanying figures, in which:
Figure 1A is a front view of a smoking substitute system in an engaged position;
Figure 1B is a front view of the smoking substitute system in a disengaged position;
Figure 1C is a section view of a smoking substitute apparatus;
Figure 2 is a front view of a smoking substitute device having a flavour delivery article; and
Figure 3 is a partial side section view of a smoking substitute apparatus having a flavour delivery article.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

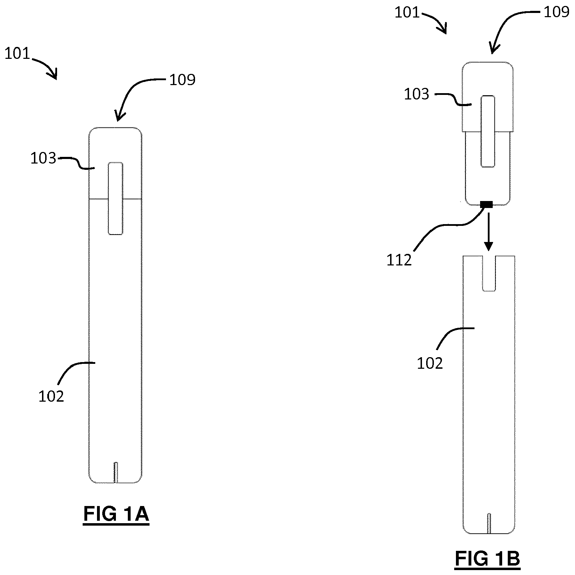

Figures 1A and 1B illustrate a smoking substitute system in the form of an e-cigarette system 101. The system 101 comprises an e-cigarette device defining a main body 102 of the system 101, and an smoking substitute apparatus in the form of an e-cigarette consumable (or "pod") 103. In the illustrated embodiment the consumable 103 (smoking substitute apparatus) is removable from the main body (e-cigarette device), so as to be a replaceable component of the system 101. In other words, the e-cigarette system 101 is a closed system.

As is apparent from Figures 1A and 1B, the consumable 103 is configured to engage the main body 102. Figure 1A shows the main body 102 and the consumable 103 in an engaged state, whilst Figure 1B shows the main body 102 and the consumable 103 in a disengaged state. When engaged, a portion of the consumable 103 is received in a cavity of the main body 102 and is retained in the engaged position by way of a snap-engagement mechanism. In other embodiments, the main body 102 and consumable 103 may be engaged by screwing one into (or onto) the other, through a bayonet fitting, or by way of an interference fit.

The system 101 is configured to vaporise an aerosol-former, which in the illustrated embodiment, is in the form of a nicotine-based e-liquid 104. The e-liquid 104 comprises nicotine and a base liquid including propylene glycol and/or vegetable glycerine. In the present embodiment, the e-liquid 104 is flavourless (and does not include any added flavourant). That is, if the e-liquid 104 were to be inhaled (i.e. in aerosol form) by a user, it would not have a particularly perceptible flavour or taste.

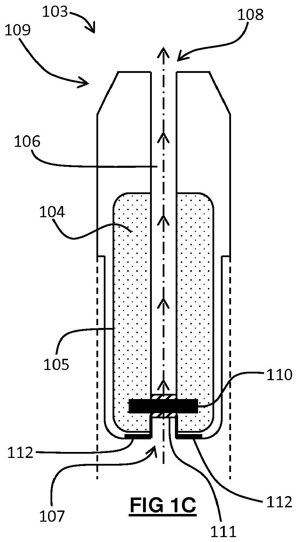

As is more apparent from Figure 1C, this e-liquid 104 is stored within a reservoir in the form of a tank 105 that forms part of the consumable 103. In the illustrated embodiment, the consumable 103 is a "single-use" consumable 103. That is, upon exhausting the e-liquid 104 in the tank 105, the intention is that the user disposes of the entire consumable 103. In other embodiments, the e-liquid (i.e. aerosol former) may be the only part of the system that is truly "single-use". That is, the tank may be refillable with e-liquid or the e-liquid may be stored in a non-consumable component of the system. For example, the e-liquid may be stored in a tank located in the main body or stored in another component that is itself not single-use (e.g. a refillable cartomizer).

The tank 105 surrounds, and thus defines a portion of, a passage 106 that extends between an inlet 107 and an outlet 108 at opposing ends of the consumable 103. In this respect, the passage comprises an upstream end at the end of the consumable 103 that engages with the main body 102, and a downstream end at an opposing end of the consumable 103 that comprises a mouthpiece 109 of the system 101. When the consumable 103 is engaged with the main body 102, a user can inhale (i.e. take a puff) via the mouthpiece 109 so as to draw air through the passage 106, and so as to form an airflow (indicated by arrows) in a direction from the inlet 107 to the outlet 108 of the passage 106. Although not illustrated, the passage 106 may be partially defined by a tube (e.g. a metal tube) extending through the consumable 103. The passage 106 is in fluid communication with a gap defined between the consumable 103 and the main body 102 (when engaged) such that air outside of the system 101 is drawn into the passage 106 (during an inhale).

The smoking substitute system 101 is configured to vaporise the e-liquid 104 for inhalation by a user. To provide this, the consumable 103 comprises a heater having of a porous wick 110 and a resistive heating element in the form of a heating filament 111 that is helically wound around a portion of the porous wick 110. The porous wick 110 extends across the passage 106 (i.e. transverse to a longitudinal axis of the passage106) and opposing ends of the wick 110 extend into the tank 105 (so as to be submerged in the e-liquid 104). In this way, e-liquid 104 contained in the tank 105 is conveyed from the opposing ends of the porous wick 110 to a central portion of the porous wick 110 so as to be exposed to the airflow in the passage 106 (i.e. caused by a user inhaling). In other embodiments the heating filament 111 and/or wick 110 may form part of the main body (but may engage the tank 105 during engagement of the main body 102 and the consumable 103).

The helical filament 111 is wound about this exposed central portion of the porous wick 110 and is electrically connected to an electrical interface in the form of electrical contacts 112 mounted at the end of the consumable that is proximate the main body 102 (when engaged). When the consumable 103 is engaged with the main body 102, the electrical contacts 112 contact corresponding electrical contacts (not shown) of the main body 102. The main body electrical contacts are electrically connected to a power source (not shown) of the main body 102, such that (in the engaged position) the filament 111 is electrically connected to the power source. In this way, power can be supplied by the main body 102 to the filament 111 in order to heat the filament 111. This heat is transferred from the filament 111 to the porous wick 110 which causes e-liquid 104 conveyed by the porous wick 110 to increase in temperature to a point at which it vaporises. The vaporised e-liquid becomes entrained in the airflow and, between the vaporisation point at the filament 111 and the outlet 108 of the passage 106, condenses to form an aerosol. This aerosol is then inhaled, via the mouthpiece 109, by a user of the system 101.

The power source of the main body 102 may be in the form of a battery (e.g. a rechargeable battery). The main body 102 may comprise a connector in the form of e.g. a USB port for recharging this battery. The main body 102 may also comprise a controller that controls the supply of power from the power source to the main body electrical contacts (and thus to the filament 111). That, is the controller may be configured to control a voltage applied across the main body electrical contacts, and thus the voltage applied across the filament 111. In this way, the filament 111 may only be heated under certain conditions (e.g. during a puff and/or only when the system is in an active state). In this respect, the main body 102 may include a puff sensor (not shown) that is configured to detect a puff (i.e. inhalation). The puff sensor may be operatively connected to the controller so as to be able to provide a signal, to the controller, which is indicative of a puff state (i.e. puffing or not puffing). The puff sensor may, for example, be in the form of a pressure sensor or an acoustic sensor.

Although not shown, the main body 102 and consumable 103 may comprise a further interface which may, for example, be in the form of an RFID reader, a barcode or QR code reader. This interface may be able to identify a characteristic (e.g. a type) of a consumable 103 engaged with the main body 102. In this respect, the consumable 103 may include any one or more of an RFID chip, a barcode or QR code, or memory within which is an identifier and which can be interrogated via the interface.

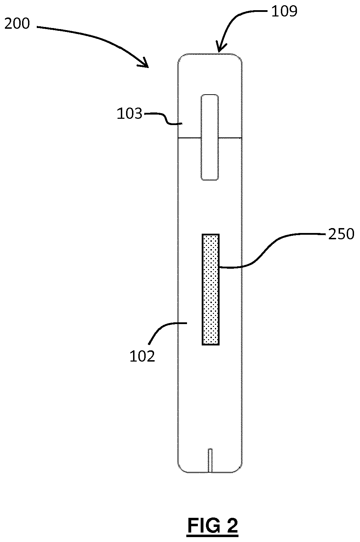

Figure 2 illustrates a smoking substitute device 200 similar to that shown in Figures 1A-1C. However, smoking substitute device 200 additionally comprises a flavour delivery article 250 for delivering flavour to a user during vaping. The flavour delivery article 250 is attached to an outer surface of the main body 102 of the smoking substitute device 200. The flavour delivery article 250 sits in a recess formed in the main body 102, and extends away from the main body 102. In an alternative embodiment, the flavour delivery article 250 is flush with the outer surface of the main body 102 so that it does not extend away from the main body 102.

Similarly to the smoking substitute system as described in Figures 1A-1C, one end of the consumable 103 of smoking substitute device 200 defines a mouthpiece 109 for delivering vaporised e-liquid 104 to a user during vaping. The flavour delivery article 250 is positioned such that during vaping, the flavour delivery article 250 is adjacent to, or proximate to, the nose of the user. In other words, the flavour delivery article 250 is positioned on the top side of the smoking substitute device 200 to sit close to the nose of a user. Accordingly, when a user inhales during vaping, vaporised e-liquid 104 containing nicotine is inhaled via the user's mouth, and flavour is inhaled via the user's nose by olfactory flavour delivery.

The flavour delivery article 250 is attachable to and detachable from the main body 102 by a clip (although other means of reversible attachment of the flavour delivery article to the main body may be used). In an alternative embodiment, the flavour delivery article 250 may be attachable to the consumable 103 instead of the main body 102.

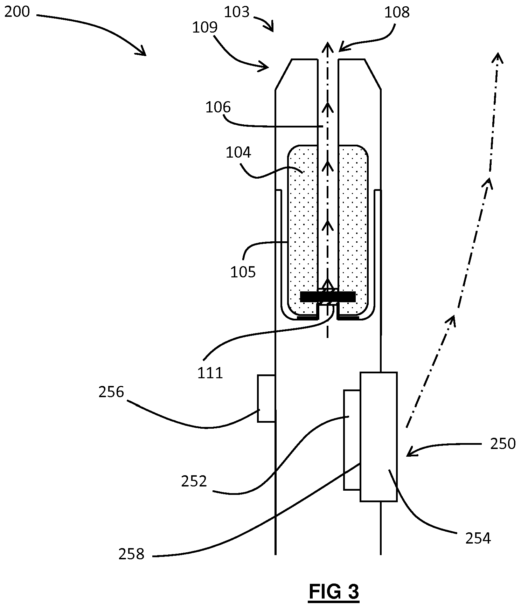

As shown in Figure 3, the flavour delivery article 250 comprises a piezoelectric motor 252 and a pad of absorbable material 254. A fragranced liquid (i.e. a scented liquid) is absorbed into the pad 254. The piezoelectric motor 252 is positioned adjacent to the pad 254. Specifically, a resonating surface 258 of the piezoelectric motor 252 is positioned adjacent to and in contact with the pad 254 containing the absorbed fragranced liquid.

Although not shown in Figure 3, both the piezoelectric motor 252 and filament 111 are connectable to a power source of the main body 102. As described above with reference to Figure 1C, main body electrical contacts are electrically connected to the power source of the main body 102, such that (in the engaged position) the filament 111 is electrically connected to the power source. In this way, power can be supplied by the main body 102 to the filament 111 in order to heat the filament 111 and vaporise the e-liquid 104 for inhalation by a user. Similarly, the main body electrical contact also electrically connects the piezoelectric motor 252 to the power source of the main body 102 (in the engaged position) so that power can be supplied from the main body 102 to the piezoelectric motor 252.

A controller (not shown) in the main body 102 controls the supply of power from the power source to the filament 111 and the piezoelectric motor 252. The controller is configured to control a voltage across the filament 111 and across the piezoelectric motor 252.

If the controller instructs the supply of power to the piezoelectric motor 252, a high frequency signal is supplied to the piezoelectric motor 252. A piezoelectric element formed from a piezoelectric material in the piezoelectric motor 252 converts this signal to vibrationary motion at the same high frequency and vibrates the resonating surface 258 at this same high frequency. As the resonating surface 258 is positioned adjacent to, and touching, the pad 254 containing the absorbed fragranced liquid, the resonating surface 258 vibrates at the same high frequency against the pad 254. The force on the pad 254 resulting from the high-frequency oscillation of the resonating surface 258 against the pad 254, leads to agitation of the fragranced liquid absorbed within the pad 254. This excitement of the fragranced liquid results in the formation of fine droplets of fragranced liquid which are released from the pad 254 as a mist.

As shown by the arrows from the pad 254 in Figure 3, the mist of fragranced liquid travels towards the nose of the user (during vaping, when the user has the mouthpiece 109 in their mouth). Accordingly, e-liquid aerosol enters the mouth of the user through the mouthpiece 109 for the delivery of nicotine, and the mist of fragranced liquid enters the nose of the user for the delivery of flavour.

The controller controls the supply of power from the power source to the piezoelectric motor 252. A user can directly control the supply of power by a dial 256 on the main body 103. Movement of the dial 256 results in the controller either increasing or decreasing the amount of power, or the frequency of the signal, supplied to the piezoelectric motor 252. Accordingly, the amplitude and/or frequency of vibration of the resonating surface 258 of the piezoelectric motor 252 can be altered, and the amount of fragranced liquid released from the pad 254 can be altered. The user can therefore control the intensity and/or strength of flavour received from the flavour delivery article 250.

In an alternative embodiment, a user may be able to control the supply of power to the piezoelectric motor 252 by a button, slider or other means.

The controller may also control the supply of power to the piezoelectric motor 252 on the basis of whether or not a user is puffing on the smoking substitute device 200. The main body 102 includes a puff sensor (not shown) that is configured to detect a puff (i.e. inhalation) on the smoking substitute device 200. The puff sensor is operatively connected to the controller so as to be able to provide a signal, to the controller, which is indicative of a puff state (i.e. puffing or not puffing). The puff sensor may, for example, be in the form of a pressure sensor or an acoustic sensor. The controller also controls power supply to the piezoelectric motor 252 in response to a puff detection by the puff sensor. Specifically, the controller may only supply power to the piezoelectric motor 252 in response to a puff detection signal indicating a user puff, such that flavour is only released when a user is taking a puff (i.e. sucking on) the smoking substitute device.

In this way, the flavour delivery article 250 is only activated to release flavour when a user is taking a puff (i.e. inhaling through) the smoking substitute device 200.

In alternative embodiments, the flavour delivery article 250 may comprise a pocket containing the fragranced fluid, or a crystalized membrane, instead of the absorbable pad 254. The dial 256 for controlling the power supplied to the piezoelectric motor 252 may be formed on the flavour delivery article 250, consumable 103 or the main body 102.

In a further alternative embodiment, the flavour delivery article 250 may be integral with the main body 102 or the consumable 103, such that the flavour delivery article 250 is not detachable from the main body 102 or consumable 103.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. A flavour delivery article for a smoking substitute device, the flavour delivery article comprising:
a flavourant source comprising a fragranced fluid; and
a flavour generator configured to agitate the fragranced fluid to thereby release the fragranced fluid from the flavour delivery article for olfactory flavour delivery.

2. The flavour delivery article of claim 1, wherein the flavour generator is configured to agitate the fragranced fluid in response to a user input.

3. The flavour delivery article of claim 1 or claim 2, wherein the flavour generator is electrically powered.

4. The flavour delivery article of claim 3, wherein an amount of electrical power supplied to the flavour generator is adjustable to modify the amount of fragranced fluid released from the flavour delivery article.

5. The flavour delivery article of any preceding claim, wherein the flavour generator comprises a piezoelectric motor.

6. The flavour delivery article of claim 5, wherein:
the piezoelectric motor comprises a piezoelectric element and a resonating surface;
the resonating surface is positioned adjacent to the flavourant source; and
the piezoelectric element is configured to vibrate the resonating surface upon receipt of an electrical signal, the resonating surface thereby providing a force on the flavourant source to agitate the fragranced fluid.

7. The flavour delivery article of any preceding claim, wherein the flavourant source is an absorbable pad and the fragranced fluid is a fragranced liquid absorbed into the pad.

8. The flavour delivery article of any of claims 1-6, wherein the flavourant source is a pocket, and the fragranced fluid is a fragranced liquid contained in the pocket.

9. The flavour delivery article of claim 8, wherein the pocket comprises an outlet mister, the outlet mister being configured to release a mist of the fragranced liquid upon agitation of the fragranced liquid by the flavour generator.

10. The flavour delivery article of any preceding claim, further comprising attaching means for attaching the flavour delivery article to a smoking substitute device.

11. A smoking substitute device comprising:
a tank for containing a vaporisable e-liquid;
a heating element for heating the vaporisable e-liquid;
an electrical power source for providing electrical power to the heating element; and
the flavour delivery article of any preceding claim.

12. The smoking substitute device of claim 11 having opposing ends, wherein one end of the smoking substitute device defines a mouthpiece, and the flavour delivery article is configured to release the fragranced fluid towards the mouthpiece.

13. The smoking substitute device of claim 11 or claim 12, wherein the flavour generator of the flavour delivery article is a piezoelectric motor, and the electrical power source is electrically connectable to the piezoelectric motor to provide electrical power to the piezoelectric motor.

14. A smoking substitute device of claim 13, further comprising a controller for controlling the supply of electrical power to the heating element and the piezoelectric motor.

15. The smoking substitute device of claim 14, further comprising a puff sensor operatively connectable to the controller so as to be able to provide a signal to the controller that is indicative of a detected puff by a user, wherein the controller is configured to instruct the supply of electrical power to the heating element and the piezoelectric motor in response to the receipt of the signal.
